# EUROPEAN PATENT SPECIFICATION

(11) **EP 1 599 233 B1**
(45) Date of publication and mention of the grant of the patent: **25.04.2007**
(21) Application number: 04716254.0
(22) Date of filing: 02.03.2004
(51) Int. Cl.: A61L 2/07

(54) **STERILIZATION OF BECLOMETHASONE DRUG PARTICLES FOR PULMONARY DELIVERY**
STERILISIERUNG VON BECLOMETASON-ARZNEIMITTELTEILCHEN ZUR ANWENDUNG IN DER LUNGE
STERILISATION DE MEDICAMENTS GLUCOCORTICOSTEROIDES PARTICULAIRES POUR APPLICATION PULMONAIRE

(30) Priority: 04.03.2003 EP 03004726
(43) Date of publication of application: 30.11.2005
(73) Proprietor: Dompé S.P.A., I-67100 L'Aquila (IT)
(72) Inventor: Gentile, Marco, I-67100 L'Aquila (IT); Dragani Maria, Concetta, I-67100 L'Aquila (IT); Dell'Uomo, Marco, I-67100 L'Aquila (IT)
(74) Representative: Pieraccioli, Daniele
(86) International application number: PCT/EP2004/050235
(87) International publication number: WO 2004/078102

(56) References cited:
- WO-A-00/25746
- WO-A-02/41925
- WO-A-95/31964
- WO-A-99/25359
- WO-A-99/32156
- WO-A-99/36055
- US-A- 3 962 430

## Description

### Brief description of the invention

The invention relates to the steam sterilization of beclomethasone, especially in the form of beclomethasone dipropionate, for inhalation delivery.

### Background of the invention

The process for sterilizing powdered forms of water insoluble drug substance to be suspended into a sterile aqueous vehicle suitable for the pulmonary administration, such as non-electrolyte corticosteroids, glucocorticoids and the like, is still a critical process. The major problems are related to the high temperatures of the sterilization process and to the consequent thermal instability of the drug substance that frequently leads to degradation with modification of the impurities profile and of the physico-chemical characteristics of the drug.

For solid drug substances suitable for inhalation delivery to be suspended in aqueous formulations, the particle size distribution, as well as its preservation during the shelf-life of the finished product, are particularly crucial parameters.

The particle size influences, in fact, the distribution of the drug into the lung and, as a consequence, the activity and effectiveness of the drug itself. It is generally accepted that the mean diameter of the particles in a formulation for inhalation delivery must be less than 10 microns, preferably about 5 microns or less.

Solid non-electrolyte corticosteroids, steroids as well as non-steroid drugs for use in aqueous suspensions are usually sterilized in different ways, for example by exposure to gases, or by aseptic crystallisation, drug heat sterilization, or by β and γ irradiation.

The sterilizing treatment can cause adverse physical and chemical changes of the drug substance and all parameters have to be checked and investigated in the preliminary phase of the process development

In the case of drug substances intended for inhalation use, in addition to the control of the physical and chemical stability of the sterilized drug, it is then crucial to prevent any unacceptable change in particle size due to possible recrystallization of the drug, which is consequent to many known sterilization methods.

A process for the steam sterilization of solid non-electrolyte corticosteroids is described by O'Neill in US 3,962,430. The process is carried out in a saturated sodium chloride solution at the sterilizing temperature. Sodium chloride is reported to be necessary to prevent recrystallization of the solid particles and any possible change in crystal size or form of the corticosteroid upon re-cooling.

WO 00/25746 describes a process for the preparation of suspensions of drug particles for inhalation delivery and includes sterilization with gamma irradiation, stating that beclomethasone dipropionate suspensions subjected to wet steam process, under conditions similar to those reported in US 3,962,430 (121°C for 15 minutes), undergo a remarkable decrease in the content of the active ingredient with a significant increase in degradation products (about 10-11 %).

Steam sterilization of the aqueous suspensions of glucocorticoids is also reported by Karlsson (WO 99/25359) to lead to unacceptable changes in particle size. Karlsson makes reference to the wet steam method described by O'Neill in US 3,962,430 stating that such method is not suitable for suspensions of fine particles of glucocorticosteroids which are intended for inhalation because the water, and the heating and the cooling involved, produce unfavourable changes in the size of the particles.

In order to eliminate the problem of changes in particle size, Karlsson uses dry heat sterilization which is, anyway, known to be often unsuitable for solid drug compounds since it causes adverse physical and chemical changes such as discoloration and degradation of the solids even at temperatures below 120° C.

### Description of the drawings

Figure 1: Particle size distribution of the drug substance BDP batch 4 sterilised (S), not sterilised (NS) and sterilised in presence of NaCl 0.420 g/mL (S NaCl(B)) and of NaCl 0.570 g/mL (S NaCl(C)).
Figure 2: Particle size distribution of the drug substance BDP batch 5 sterilised (S), not sterilised (NS) and sterilised in presence of NaCl 0.420 g/mL (S NaCl(B)) and of NaCl 0.570 g/mL (S NaCl(C)).
Figure 3: Particle size distribution of formulation PG038/115 (BDP batch 4 sterilised) and PG038/119 (BDP batch 4 not sterilised) after 50 days of storage at 40°C 75%R.H.
Figure 4: Particle size distribution of formulation PG038/116 (S) (BDP batch 5 sterilised) and formulation PG038/120 (NS) (BDP batch 5 not sterilised) after 50 days of storage at 40°C 75%R.H.

### Detailed description of the invention

We have unexpectedly found, given the teachings of the prior art, a simple and economic process for the sterilization of beclomethasone and his salts, using an aqueous suspension of the substance and avoiding the complex and more expensive processes of the prior art. The process of the present invention is particularly suitable for beclomethasone diproprionate (BDP) (Prontinal^{®} - Dompé) and the use of said process in industrial plants allows an easier and less expensive manufacturing process. The sterilisation of the active ingredient can be performed directly in the preparation vessel (turbohomogenizer working as an autoclave); in this way the bulk preparation and the transfer of bulk to the filling machine can be carried out without any contact with the environment (aseptic condition). As a consequence, qualification and controls of preparative area, personnel gowning and training as well as cleaning procedures, result to be less heavy in terms of costs and timing.

It is thus an object of the present invention to provide a process for the steam sterilisation of beclomethasone, comprising heating a mixture of water and micronised beclomethasone at a temperature ranging between 100° and 130°C for a time sufficient to sterilise the mixture with a minimum S.A.L. (Sterility Assurance Level - European Pharmacopoeia 4, 2003, 5.1. General Texts on sterility, pag 405) of 10⁻⁶, the mixture being a mixture of beclomethasone and water only. The micronised beclomethasone:water (w:w) ratio can range between 2.5:100 and 100:2. When the ratio is 100:2 the mixture is intended to be a micronised hydrated beclomethasone powder.

Preferably the beclomethasone is in the form ofbeclomethasone diproprionate.

The micronised beclomethasone:water ratio is preferably between 3:100 (about 30mg/mL) to 10:100 (about 100mg/mL).

Mixtures of the beclomethasone and water at different ratios were prepared and the mixtures were steam sterilised at a temperature of about 120° C for a time ranging from 15 to 30 minutes.

Preferably steam sterilization was carried out at 121°C for 20 minutes.

The purity of BDP sterilised according to the present invention was compared with the purity of the starting, non-sterilised BDP.

The distribution in the particle size and the water stability of the sterilised BDP was also determined in order to verify the steam treatment effects on crystal growth.

In parallel, steam sterilization of BDP in the presence of saturated sodium chloride, according to US 3,962,430, was also carried out.

The amount of sodium chloride was in the range between 1.1 mg to 1.5 mg per 2.6 mg of water, according to the teachings of US 3,962,430. The comparative tests were then carried out with mixtures NaCl:BDP:water in the ratio 42:4:100 and 57:4:100, corresponding to an aqueous suspension containing about 40mg of BDP and 420mg or 570mg of sodium chloride in a final volume of 1mL.

The comparative steam sterilization in the presence of saturated sodium chloride was carried out at the same temperature and for the same time as the steam sterilization experiments carried out according to the present invention.

Stability studies were also performed with formulations suitable for inhalations prepared using the steam sterilised BDP suspensions of the present invention in comparison with formulations containing non-sterilized BPD.

Analyses for particle size and HPLC purity were performed on samples after 50 days of storage at 40° C, 75% R.H.

Results show that the impurities profile of the sterilised glucocorticoid suspensions of the present invention are not significantly different from the profile of the non-sterilised glucocorticoid.

Also the particle size distribution of the sterilised suspensions is not different from the particle size distribution of the non-sterilised glucocorticoid and is not different from the size of the glucocorticoid sterilised in a sodium chloride saturated solution. Sodium chloride does not affect the crystal size of aqueous beclomethasone dipropionate suspensions, contrary to what stated in the art by O'Neill (US 3,962,430).

In addition, no significant differences were found in crystal growth and size distribution between formulations prepared with steam sterilised glucocorticoid and with non-sterilised glucocorticoid, after storage under accelerated conditions, for 50 days at 40°C 75% R.H.

### Data processing

The experimental data, obtained during analytical determination were processed by means of the statistical function of Analysette 22 program version 1.70.

### HPLC purity of drug substance and drug product

The acceptance limits of impurities are defined in the monograph of the drug substances and drug product.

Impurities with content greater than 0.05% with reference to BDP content of the analysed sample are reported.

The HPLC purity of sterilised 4mg/mL and 40mg/mL aqueous BDP suspension were checked in comparison with non-sterilised BDP drug substance.

Synthesis drug substance impurities are not reported.

In table 1 the analytical data of three different batches of BDP drug substances (1, 2 and 3) steam sterilised at concentration of 4mg/mL and 40mg/mL in comparison with the respective BDP not sterilised are reported.

**Tab.1**

| | | | **BDP sample** | | |
|---|---|---|---|---|---|
| **BDP batch** | **HPLC purity (% content)** | **Limits** | **Not sterilised** | **4 mg/mL sterilised** | **40 mg/mL sterilised** |
| **1** | | | | | |
| | beclomethasone 17-propionate | ≤ 1.0% | ---- | ---- | ---- |
| | beclomethasone 21-propionate | ≤ 1.0% | 0.34 | 0.32 | 0.36 |
| | Rrt 0.60 | ≤ 0.1% | ---- | 0.26 | ---- |
| | Rrt 0.75 | ≤ 0.1% | ---- | ---- | ---- |
| | Rrt 0.86 | ≤ 0.1% | ---- | ---- | ---- |
| | Rrt 1.06 | ≤ 0.1% | ---- | 0.80 | ---- |
| 2 | beclomethasone 17-propionate | ≤ 1.0% | 0.13 | 0.09 | 0.13 |
| | beclomethasone 21-propionate | ≤ 1.0% | ---- | 0.06 | 0.08 |
| | Rrt 0.60 | ≤ 0.1% | ---- | 0.21 | ---- |
| | Rrt 0.75 | ≤ 0.1% | ---- | ---- | ---- |
| | Rrt 0.86 | ≤ 0.1% | ---- | ---- | ---- |
| | Rrt 1.06 | ≤ 0.1% | ---- | 0.47 | ---- |
| 3 | beclomethasone 17-propionate | ≤ 1.0% | ---- | ---- | ---- |
| | beclomethasone 21-propionate | ≤ 1.0% | 0.10 | 0.06 | 0.12 |
| | Rrt 0.60 | ≤ 0.1% | ---- | 0.21 | ---- |
| | Rrt 0.75 | ≤ 0.1% | ---- | ---- | 0.07 |
| | Rrt 0.86 | ≤ 0.1% | ---- | ---- | 0.06 |
| | Rrt 1.06 | ≤ 0.1% | ---- | 0.57 | ---- |

Sterilised 4mg/mL BDP samples show two different unknown impurities (Rrt 0.60 and Rrt 1.06) not found in the respective starting drug substance. The content of these impurities is respectively about 0.3% and 0.8%. In the samples 40mg/mL the content of these unknown impurities is below 0.05% (according to the international guideline CPMP/ICH/2737/99 "Note for guidance on impurities testing: impurities new drug substances" the reporting threshold for impurity to be identified and qualified is 0.1%). The content of the other known impurities is not significantly modified by the steam sterilisation process. This evidence is confirmed by data reported in tab. 2 where the purity profile of sterilised 40mg/mL BDP suspension, batches 4 and 5 (different experimental set), is comparable with the purity of the BDP starting drug substances.

**Tab. 2**

| **HPLC Purity** (% content) | **limits** | **40mg/ml BDP (batch 4)** | | **40mg/ml BDP (batch 5)** | |
|---|---|---|---|---|---|
| | | Not sterilized | Sterilized | Not sterilized | Sterilized |
| beclomethasone | ≤ 1.0% | ---- | ---- | ---- | ---- |
| beclomethasone 17-propionate | ≤ 1.0% | ---- | ---- | ---- | --- |
| beclomethasone 21-propionate | ≤ 1.0% | 0.13 | 0.12 | ---- | ---- |
| Rrt 0.37 | ≤ 0.1% | ---- | ---- | ---- | ---- |
| Rrt 0.62 | ≤ 0.1% | ---- | ---- | ---- | ---- |
| Rrt 1.06 | ≤ 0.1% | ---- | ---- | ---- | ---- |

The HPLC purity of formulations was determined on the formulation reported below:
- PG03 8/115 containing sterilised BDP batch 4
- PG038/119 containing not sterilised BDP batch 4
- PG038/116 containing sterilised BDP batch 5
- PG038/120,containing not sterilised BDP batch 5
The analyses was performed after the preparation (time zero T0) and after 50 days of storage at condition of 40°C 75% R.H. As reported in table 3 and 4 the purity of formulations containing sterilised BDP is not significant different from the purity of formulations containing the respective not sterilised BDP. The impurity content determined at T0 is non significantly different from the content determined after 50 days of storage at condition of 40°C 75% R.H.

**Tab. 3**

| | | **Formulations** | | | |
|---|---|---|---|---|---|
| **HPLC Purity** (% content) | **limits** | **PG038/115** (sterilised BDP 4) | | **PG038/119** (not sterilised BDP 4) | |
| | | T0 | 50 days | T0 | 50 days |
| beclomethasone | ≤ 1.0% | ---- | 0.08 | ---- | 0.08 |
| beclomethasone 17-propionate | ≤ 1.0% | ---- | 0.10 | ---- | 0.09 |
| beclomethasone 21-propionate | ≤ 1.0% | 0.12 | 0.09 | 0.12 | 0.09 |
| Rrt 0.37 | ≤ 0.5% | ---- | 0.07 | ---- | 0.09 |
| Rrt 0.62 | ≤ 0.5% | ---- | 0.15 | ---- | 0.12 |
| Rrt 1.06 | ≤ 0.5% | ---- | ---- | ---- | ---- |

**Tab. 4**

| | | **Formulations** | | | |
|---|---|---|---|---|---|
| **HPLC Purity** (% content) | **limits** | **PG038/116** (sterilised BDP 5) | | **PG038/120** (not sterilised BDP 5) | |
| | | T0 | 50 days | T0 | 50 days |
| beclomethasone | ≤ 1.0% | ---- | 0.07 | ---- | ---- |
| beclomethasone 17-propionate | ≤ 1.0% | ---- | 0.10 | ---- | 0.12 |
| beclomethasone 21-propionate | ≤ 1.0% | ---- | ---- | ---- | ---- |
| Rrt 0.37 | ≤ 0.5% | ---- | ---- | ---- | 0.09 |
| Rrt 0.62 | ≤ 0.5% | ---- | 0.19 | ---- | 0.13 |
| Rrt 1.06 | ≤ 0.5% | ---- | 0.06 | ---- | 0.09 |

### Water stability test of drug substance

Water stability test was performed on sterilised 40mg/mL BDP suspension and data were compared to not sterilised BDP. The analysed BDP batches were batch 4 and batch 5.

No substantial differences on crystal shape and size were found between sterilised samples and not.

### Particle size distribution of drug substance

Particle size, x-axis, is expressed as volume equivalent diameter (dᵥ). According to this definition each irregular particle is assumed to be a sphere. Such sphere is so defined *equivalent sphere.* Such sphere and the irregular particle have the same volume, therefore the volume of the sphere is defined as *volume equivalent sphere,* and the diameter of such *volume equivalent sphere* is defined as *volume equivalent diameter*. A log scale is reported.

The number of particles within each sizeclasses was reported on the y-axis.

The particle size distribution was determined on sterilised 40mg/mL aqueous BDP suspension. Two different batches of drug substances were investigated: batch 4 and batch 5. In addition the particle size distribution of the same batches sterilised in presence of two different concentration of NaCl (0.420 g/mL and 0.570 g/mL, referred to as NaCl (B) and

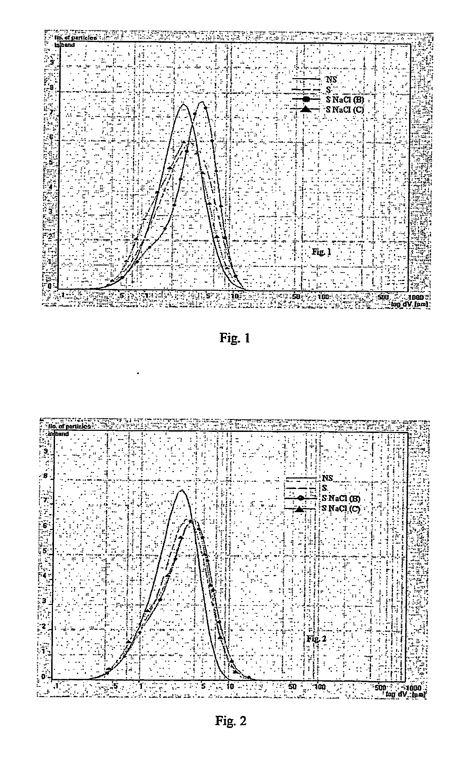

NaCl (C) respectively) were performed. The particle size distribution of samples containing BDP batch 4 and BDP batch 5 are respectively reported in figure1 and figure 2. Analyses of sterilised BDP were performed on the sample described in Example 2.

For each particle size distribution, the percentage of particles below 5 and 10 µm and the D[4,3] (equivalent volume mean diameter) were determined. D[4,3] value is an index of the particles mean diameter considering the equivalent volume and mass of the particle. The results are reported respectively in table 5 for batch 4 and table 6 for batch 5.

**Tab. 5**

| | | **BDP batch 4** | | | |
|---|---|---|---|---|---|
| | | not sterilised | | sterilised + NaCl (0.42 g/mL) | sterilised + NaCl (0.57 g/mL) |
| D[4.3] | | 2.80 µm | 3.27 µm | 3.79 µm | 2.9 µm |
| Particle size | 5 µm | 90.66 % | 81.54 % | 74.91 % | 87.09 % |
| | < 10 µm | 99.48 % | 98.91 % | 98.77 % | 98.99 % |

**Tab. 6**

| | | **BDP batch 5** | | | |
|---|---|---|---|---|---|
| | | not sterilised | | sterilised + NaCl (0.42 g/mL) | sterilised + NaCl (0.57 g/mL) |
| D[4.3] | | 2.73 µm | 3.38 µm | 3.75 µm | 3.37 µm |
| Particle size | < 5 µm | 92.05 % | 81.33 % | 76.60 % | 80.63 % |
| | < 10 µm | 99.98 % | 98.19 % | 97.47 % | 98.67 % |

As shown in figures 1 and 2 and confirmed by the value referring to D[4.3] (tables 5 and 6) the sterilisation process promoted a slight crystal growth of BDP. The D[4,3]value of sterilised BDP was about 0.6 micron greater than the not sterilised. The amount of particle with a mean diameter less than 5 micron (n.b. particle size below 5 micron is considered to be the respirable fraction) was respectively around 80% for sterilised BDP and 90% for the not sterilised BDP. The amount of particles with a mean diameter less than 10 micron was always greater than 98%. Contrary to what it is reported by O'Neill (US 3962430) a growth of crystal size to 300-400 micron, subsequent to the sterilisation process, was not observed. The presence of NaCl at the two concentration (pre-mixed in the BDP suspension to be sterilised) did not substantially affect the particle size. The particle size profile of sterilised samples containing BDP + NaCl or BDP alone were not significantly different. Only the curve of sterilised BDP+0.42 mg/mL of NaCl (fig. 1) was slightly shifted towards the right (increase crystal size) in comparison with the curve of the sterilised BDP.

### Particle size distribution of drug product

Particle size distribution of each formulation was determined for comparison. The compared formulations had the same quali-quantitative composition of excipients (see tab. 9), but contained sterilised BDP or not sterilised BDP as reported in tab. 7.

**Tab.7**

| ***Batch formulation*** | ***Batch BDP*** | ***Characteristic of the batch*** |
|---|---|---|
| PG038/115 | 4 | Sterilised |
| PG038/116 | 5 | Sterilised |
| PG038/119 | 4 | Not sterilised |
| PG038/120 | 5 | Not sterilised |

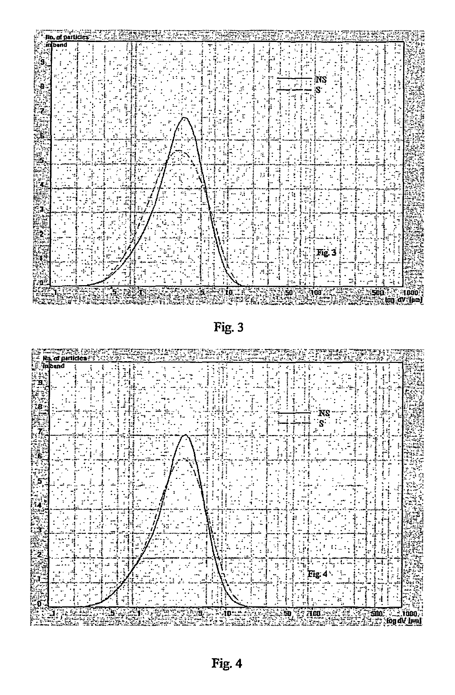

Analyses were performed on the sample after 50 days of storage at 40°C 75% R.H. Figure 3 shows particle size distribution of formulations PG038/115(S) and PG038/119(NS) Figure 4 shows particle size distribution of formulations PG038/116(S) and PG038/120(NS).

The D[4,3]value and the percentage of particle with a dᵥ lower than 5 and 10 µm of each analysed samples are reported in table 8.

**Tab. 8**

| | | **PG038/119** (not sterilised BDP 4) | **PG038/115** (sterilised BDP 4) | **PG038/120** (not sterilised BDP 5) | **PG038/116** (sterilised BDP 5) |
|---|---|---|---|---|---|
| D [4.3] Particle size | | 324 µm | 3.21 µm | 3.41 µm | 3.53 µm |
| | < 5 µm | 83.76 % | 81.79 % | 81.65 % | 78.48 % |
| | < 10 µm | 99.13 % | 98.62 % | 98.64 % | 97.85 % |

The curve profiles of formulations containing sterilised BDP and the respective not sterilised BDP are not significantly different. The D[43] and the amount of particles with a diameter less than 5 µm, of all formulations, were not affected by the characteristic of the used BDP. The D[4.3] value of formulation containing sterilised BDP was the same of the formulation containing not sterilised BDP. The percentage of particle with a diameter less than 5 micron was comparable in both formulations.

Similar results have been obtained with formulations containing steam sterilised BDP: water mixtures in all investigated ratios (range 2.5:100 - 100:2), for example mixtures containing 400 mg BDP and 1 ml water or mixtures containing 4g BDP and 1 ml water or even mixtures containing 40g BDP and 1 ml water.

A slight crystal growth is observed in the sterilised samples of drug substance, but the diameter of particles remains always below 20-30micron and no particles are observed with a size around 300-400micron, contrary to what stated in US 3,962,430. A saturated solution of sodium chloride, used instead of water only, doesn't affect the crystal size, so that the particle size distribution of sterilised BDP is not significantly different from the particle size distribution of BDP sterilised in the presence of sodium chloride.

The particle size distribution of formulations containing sterilised BDP and non-sterilised BDP respectively, is comparable after 50 days in samples stored at stressed condition, and no significant differences were found between the different formulations.

It has also been found that micronized aqueous suspensions of glucocorticoids can be efficiently steam sterilised under the above conditions without any modification of the purity of the drug substance. This result was also unexpected after the teachings of WO 00/25746, which reports that BDP suspensions subjected to a steam sterilisation process undergo a remarkable degradation amounting to about 8-9% of the active ingredient.

The steam sterilization of glucocorticoids according to the present invention is, therefore, the simplest and most economic process to be used in industrial plants for the preparation of pharmaceutical formulations of glucocorticoids, in particular of BDP, for inhalation delivery.

The process for the sterilisation of glucocorticoid, and beclomethasone dipropionate in particular, developed with the present invention offers therefore, considerable economic advantages in the development of an industrial aseptic process for a sterile glucocorticoid manufacturing in a formulation suitable for pulmonary delivery.

The present invention shall be illustrated by means of the following examples which are not construed to be viewed as limiting the scope of the invention.

### Materials and Methods

| **Materials** | |
|---|---|
| | ***Supplier*** |
| Beclomethasone dipropionate | Farmabios |
| Sorbitan laurate | IVI |
| Polysorbate 20 | IVI |
| Cetostearyl alcohol | IVI Chimica |
| Sodium monohydrogen phosphate bihydrate | Fluka |
| Potassium dihydrogen phosphate | Fluka |

| **Equipment** | |
|---|---|
| Autoclave: | De Lama mod. VS/E |
| Stirrer: | IKA RW 20 DZM |
| Homogenizer: | IKA Ultra Turrax T 50 |
| Particle size analyser: | Fritsch Analysette A22 |

### EXAMPLE 1

### Steam sterilisation of aqueous BDP suspension.

Two different concentrations, 4mg/mL and 40mg/mL were used. The steam sterilisation was performed in autoclave at 121°C 20'. Three different batches of BDP were used hereinafter referred to as batch 1, batch 2 and batch 3.

The HPLC purity of sterilised DBP was evaluated in comparison to the non-sterilised BDP. Each batch has prepared as follows:
BDP (4 mg) was suspended in 1 mL of purified water
BDP (40 mg) was suspended in 1 mL of purified water.

### EXAMPLE 2

### Particle size distribution and water stability test of sterilised BDP

Particle size distribution and water stability test were performed on sterilised BDP and, for comparison with non-sterilised BDP. BDP was sterilised as aqueous 40mg/mL suspension.

In addition aqueous 40mg/mL BDP suspensions containing 420mg/mL and 570mg/mL of NaCl were sterilised. In both cases the steam sterilisation was carried out at 121°C 20'.

Two different batches of BDP were used (batch 4 and batch 5), of each preparation.
***BDP batch 4 and BDP batch 5:*** 120 mg of BDP was suspended in 3 mL of purified water.
***BDP batch 4* + *NaCl (B) and BDP batch 5* + *NaCl(B):*** 120 mg of BDP and 1.26 g of NaCl were suspended in 3 mL of purified water
***BDP batch 4* + *NaCl(C) and BDP batch 5* + *NaCl(C):*** 120 mg of BDP and 1.71 g of NaCl were suspended in 3 mL of purified water

The particle size distribution curves were obtained using a laser particle sizer "Fritsch Analysette 22".

### EXAMPLE 3

### Formulation for stability study

Formulations containing sterilised BDP were prepared using the BDP samples described in Example 2.

Formulation batch number and the characteristic of BDP are reported in tab.7.

Particle size distribution was determined after 50 days of storage.

HPLC purity was evaluated immediately after the preparation and after 50 days of storage at 40°C 75% R.H.

The quali-quantitative composition of the formulation are reported in Tab. 9.

**Tab. 9**

| ***Ingredient*** | ***Amount*** |
|---|---|
| BDP | 40 mg |
| Sorbitan laurate | 20 mg |
| Polysorbate 20 | 100 mg |
| Sodium monohydrogen phosphate bihydrate | 724 mg |
| Potassium dihydrogen phosphate | 354 mg |
| Sodium chloride | 420 mg |
| Purified water | Up to 100 mL |

Following is an exemplary method for the preparation of the BDP formulation of Table 9, which was carried out in 3 distinct phases:

### Phase 1

Sorbitan laurate (0.180 g) and polysorbate 20 (0.900 g) were added to 400 mL of purified water. Then 9 mL of sterilised aqueous BDP suspension, corresponding to a total amount of 360mg of BDP, was added.

### Phase 2

Sodium monohydrogen phosphate bihydrate (6.516 g), potassium dihydrogen phosphate (3.186 g) and sodium chloride (3.78 g) were dissolved in 200 mL of purified water.

### Phase 3 - Final formulation

The preparations of phase 1 and 2 were mixed and diluted to 900 mL with purified water

Each final formulation was packaged into a 200 mL brownish glass bottle.

The formulations were submitted to stability studies under the accelerated condition of 40°C 75% R.H.

Other formulations were prepared to confirm the applicability of the process of the present invention.

The quali-quantitative composition of other formulations which have been tested is reported in Tab. 10 and Tab. 11.

**Tab. 10**

| ***Ingredient*** | ***Amount*** |
|---|---|
| BDP | 40 mg |
| Sorbitan laurate | 20 mg |
| Polysorbate 20 | 100 mg |
| Sodium chloride | 900 mg |
| Purified water | Up to 100 mL |

**Tab.11**

| ***Ingredient*** | ***Amount*** |
|---|---|
| BDP | 40 mg |
| Sorbitan laurate | 20 mg |
| Polysorbate 20 | 100 mg |
| Cetostearyl alcohol | 130 mg |
| Sodium monohydrogen phosphate bihydrate | 724 mg |
| Potassium dihydrogen phosphate | 354 mg |
| Sodium chloride | 420 mg |
| Purified water | Up to 100 mL |

## Claims

1. A process for the steam sterilization of beclomethasone comprising heating a mixture of micronized beclomethasone and water at a temperature ranging between 100 and 130° C for a time sufficient to sterilise the mixture with a minimum Sterility Assurance Level (S.A.L.) of 10⁻⁶, the mixture being a mixture of beclomethasone and water only.

2. A process according to Claim 1, wherein beclomethasone is in the form of beclomethasone dipropionate.

3. A process according to Claims 1 and 2, wherein the beclomethasone:water ratio is selected in a range between 3:100 to 10:100

4. A process according to any of Claims 1 to 3, wherein sterilization is carried out at a temperature of 121°C for 20 minutes.

## Patentansprüche

1. Verfahren zur Dampfsterilisation von Beclomethason, bestehend aus dem Erhitzen einer Mischung aus mikronisiertem Beclomethason und Wasser in einem Temperaturbereich zwischen 100 und 130°C über einen Zeitraum, der ausreicht, die Mischung mit einem minimalen Sterilitätssicherheitswert von 10⁻⁶ zu sterilisieren, wobei die Mischung nur aus Beclomethason und Wasser besteht.

2. Verfahren nach Anspruch 1, bei dem Beclomethason in Form von Beclamethason-Diproprionat verwendet wird.

3. Verfahren nach Ansprüche 1 oder 2, bei dem für das Verhältnis aus Beclomethason und Wasser ein Bereich zwischen 3:100 und 10:100 gewählt wird.

4. Verfahren nach einem der Ansprüche 1 - 3, bei dem die Sterilisation bei einer Temperatur von 121°C über 20 Minuten erfolgt.

## Revendications

1. Un procédé pour la stérilisation à vapeur de la béclométhasone comprenant le chauffage d'un mélange de béclométhasone micronisée et d'eau à une température variant entre 100 et 130 °C pour un laps de temps suffisant à stériliser le mélange avec un minimum de niveau d'assurance de stérilité de 10⁶, le mélange étant composé uniquement du mélange de béclométhasone et d'eau.

2. Un procédé selon la revendication 1, dans lequel la béclométhasone est sous la forme de dipropionate de béclométhasone.

3. Un procédé selon les revendications 1 et 2, dans lequel le rapport béclométhasone/eau est sélectionné dans une fourchette entre 3/100 à 10/100.

4. Un procédé selon les revendications 1 à 3, dans lequel la stérilisation a lieu à une température de 121 °C pendant 20 minutes.
